(19) Europäisches Patentamt
European Patent Office
Office européen des brevets

(11) Numéro de publication: **0 356 321 A1**

(12) **DEMANDE DE BREVET EUROPEEN**

(21) Numéro de dépôt: **89402299.5**

(22) Date de dépôt: **17.08.89**

(51) Int. Cl.5: **C 07 D 295/02**
C 07 D 295/12,
C 07 D 235/30,
C 07 D 249/14,
C 07 D 251/48,
C 07 D 251/54,
C 07 D 211/14, C 08 K 5/18,
C 08 K 3/16

(30) Priorité: **24.08.88 FR 8811184**

(43) Date de publication de la demande:
**28.02.90 Bulletin 90/09**

(84) Etats contractants désignés:
**AT BE CH DE ES FR GB GR IT LI LU NL SE**

(71) Demandeur: **ATOCHEM**
**4 & 8, Cours Michelet La Défense 10**
**F-92800 Puteaux (FR)**

(72) Inventeur: **Sallet, Daniel**
**13, rue Dupont de l'Eure**
**F-27470 Serquigny (FR)**

**Deloy, Pierre**
**12, Allée Youri Gagarine**
**F-92300 Levallois-Perret (FR)**

**Mailhos-Lefievre, Valérie**
**14, rue Fauvet**
**F-75018 Paris (FR)**

**Poisson, Pierre**
**Le Mascrier**
**F-27300 Bernay (FR)**

(54) **Compositions ignifugeantes pour résines synthéthiques à base de composés halogéno-métalliques azotés et résines synthétiques renfermant lesdits composés.**

(57) L'invention concerne une composition ignifugeante pour résines synthétiques, comprenant au moins un sel organo-métallique renfermant des groupements cationiques azotés et des groupements anioniques halogéno-métalliques.

L'invention concerne également de nouveaux composés halogéno-métalliques azotés.

EP 0 356 321 A1

## Description

## COMPOSITIONS IGNIFUGEANTES POUR RESINES SYNTHETIQUES A BASE DE COMPOSES HALOGENO-METALLIQUES AZOTES ET RESINES SYNTHETIQUES RENFERMANT LESDITS COMPOSES

La présente invention a pour objet une composition utilisable notamment pour l'ignifugation de résines synthétiques comprenant au moins un sel organo-métallique, renfermant des groupements cationiques azotés et des groupements anioniques halogéno-métalliques.

Pour de nombreuses applications il est indispensable de disposer de matériaux synthétiques ayant un comportement au feu amélioré.

Pour cela on préconise l'emploi de divers additifs organiques halogénés tels que les paraffines chlorées, les polybromodiphényléthers...

On a également proposé dans le brevet US 3 643 311 l'emploi de sels inorganiques tels que $(NH_4)_2\,TiF_6$ pour ignifuger les résines synthétiques telles que le polystyrène, le polypropylène ou les résines ABS.

Afin d'obtenir un niveau d'efficacité suffisant il est nécessaire d'associer à ces composés organiques ou inorganiques des oxydes métalliques tels que le trioxyde d'antimoine ou le trioxyde de bismuth.

Ceci présente l'inconvénient d'utiliser d'une façon générale des pourcentages importants d'additifs, qui peuvent atteindre 30 à 40 % du poids de la composition et qui sont de nature à abaisser les propriétés mécaniques des matériaux ignifugés.

Il a maintenant été trouvé une composition ignifugeante de résines synthétiques, efficace à faible dose, cette composition contenant au moins un composé organo-métallique renfermant des groupements cationiques azotés et des groupements anioniques halogéno-métalliques, répondant à la formule générale :

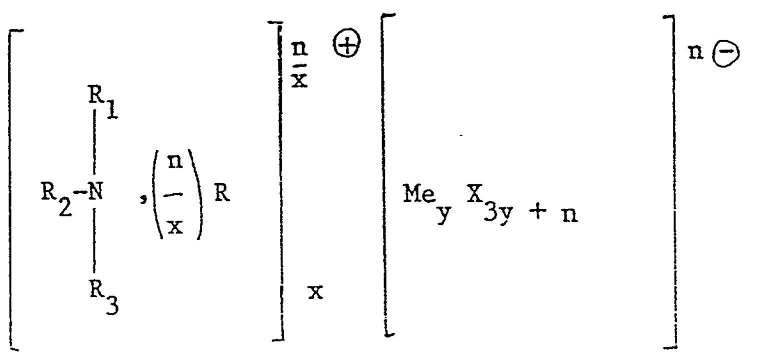

I

dans laquelle :
R, $R_1$, $R_2$ et $R_3$, identiques ou différents, représentent des atomes d'hydrogène, des radicaux hydrocarbonés aliphatiques linéaires ou ramifiés, éventuellement substitués par des halogènes et ayant jusqu'à 12 atomes de carbone, des radicaux cycloaliphatiques, des radicaux phényles substitués ou non par des halogènes, des groupements amino, des groupements aliphatiques linéaires ou ramifiés ayant jusqu'à 6 atomes de carbone ;
$R_1$, $R_2$ et $R_3$ peuvent former ensemble avec l'atome d'azote un hétérocycle aromatique substitué ou non par des groupements aliphatiques linéaires ou ramifiés ayant jusqu'à 6 atomes de carbone ou des halogènes ;
$R_2$ et $R_3$ peuvent également former ensemble avec l'atome d'azote et le groupement divalent (Y) un hétérocycle ayant de 2 à 10 atomes de carbone de formule :

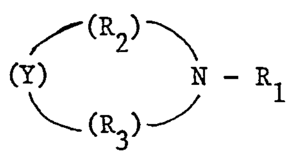

dans laquelle (Y) désigne :
le lien valentiel,

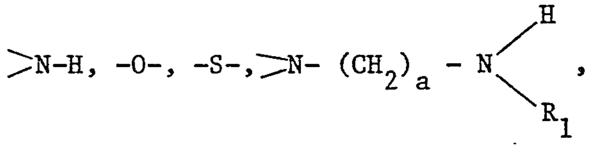

avec a compris entre 2 et 6, $R_1$ ayant la signification donnée précédemment ;
$R_3$ peut représenter un radical hétérocyclique azoté substitué ou non par des halogènes, des groupements amino, des radicaux aliphatiques linéaires ou ramifiés ayant jusqu'à 10 atomes de carbone, des radicaux phényles ou un reste amino de formule

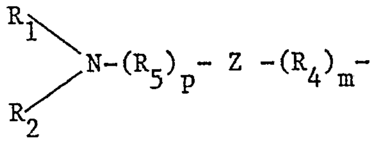

dans laquelle $R_1$ et $R_2$ ont la signification donnée précédemment, $R_4$ et $R_5$, identiques ou différents, représentent des radicaux divalents hétérocycliques azotés ou bien des restes méthyléniques,
Z représente une simple liaison ou un groupement divalent pris parmi les suivants :

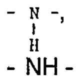

- NH - $(CH_2)_b$ NH - avec b compris entre 0 et 6,
- $(CH_2)_c$ avec c compris entre 1 et 6 et

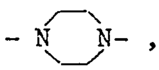 ,

p et m sont compris entre 1 et 6 ;
Me représente un métal tel que l'arsenic (III), l'antimoine (III) ou le bismuth (III) ;
X représent un halogène tel que le brome, le chlore ou l'iode
x, y et n sont des nombres entiers compris entre 1 et 10 avec $n \geqq x$

On connait déjà certains halogéno antimonates et halogéno-bismuthates de formule I, à savoir ceux dans la formule desquels le groupement cationique est

$$\left[NH_4\right]^{\oplus}_x$$

décrits par G Jander (Z. Electrochim, 61, 1275 1957) et par L.P Pandey (J. Indian Chem. Soc 41 (11) 771.3 1964) ou bien ceux dans la formule desquels le groupement cationique est un radical alkylammonium, alkyldiammonium, anilinium, cyclohexylammonium, pipéridinium décrits par R.D. WHEALY et R.L. YEAKLEY (J. Inorg. Nucl. Chim. 25, 365 - 368, 1963) et par M.A HOOPER et D.W JAMES (Aus. J. Chem, 26,149 - 12, 1973) ou bien pyridinium et quinoléinium décrits par J.M STEWART et collaborateurs (Inorganic Chemistry, 13 (11) 2767, 1974).

On connait également des composés de formule I dans la formule desquels le groupement cationique est un tetralkyl ammonium décrits par G.Y. AHLIJAH et M. GOLDSTEIN (J. Chem. Soc (A), 326 - 328, 1970).

Aucune des références citées n'a décrit ou suggéré l'utilisation de ces produits dans des compositions ignifugeantes.

Les autres composés sont des produits nouveaux de formule

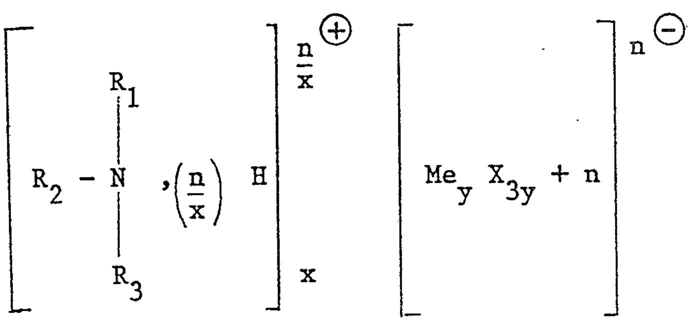 (I - a)

et, comme tels font partie de la présente invention.

Dans cette formule $R_1$ et $R_2$, identiques ou différents, représentent des atomes d'hydrogène, des radicaux aliphatiques linéaires ou ramifiés ayant jusqu'à 12 atomes de carbone, des radicaux phényles ;
$R_3$ représente un radical hétérocyclique azoté, C-substitué ou non, par des halogènes, des groupements amino, des radicaux aliphatiques linéaires ou ramifiés ayant jusqu'à 10 atomes de carbone, des radicaux phényles ou un reste amino de structure $H_2N$ - $(R_5)_p$- Z $(R_4)_m$- dans lequel $R_4$ et $R_5$ identiques ou différents, représentent des radicaux divalents hétérocycliques, Z est un groupement divalent pris parmi les suivants :

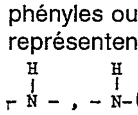-$(CH_2)_b$- NH avec b compris entre 0 et 6,

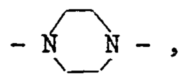

ou un reste - $(CH_2)$-$_c$ avec c compris entre 1 et 6 lorsque $R_5$ et $R_4$ représentent des radicaux divalents hétérocycliques, p et m sont compris entre 1 et 6 ;
$R_2$ et $R_3$ peuvent former également ensemble avec l'atome d'azote et les groupements divalents N - H ou N - $(CH_2)_a$- $NH_2$ avec a compris entre 2 et 6, un reste hétérocyclique ayant de 2 à 10 atomes de carbone;
Me, X, n, x et y sont tels que définis précédemment.

D'une façon générale les composés de formule I peuvent être préparés suivant des méthodes connues en soi. On peut, par exemple, utiliser la technique décrite dans les articles de WHEALY et R.L. YEAKLEY (op. cit) et de G. C Allen et R.F. Mc Meeking (Inorganica Chimica Acta, 23, 185 - 190, 1977) et qui consiste à faire réagir un halogénure d'ammonium sur un halogénure métallique.

Une autre méthode consiste à faire réagir une amine avec un halogénure métallique en solution halohydrique (voir par exemple l'article de J.M STEWART) (op. cit)

Les composés de formule (I - a) selon notre invention ont été préparés selon des méthodes similaires à celles mentionnées ci-dessus.

Ainsi selon une première variante, on fait réagir une amine avec un hydracide halogéné puis on complexe l'halogénure d'ammonium substitué obtenu par un halogènure métallique en solution halohydrique selon les réactions :

a) 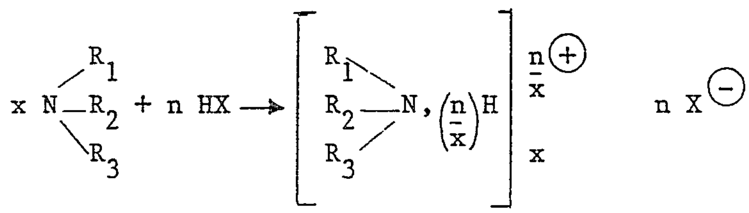

b) 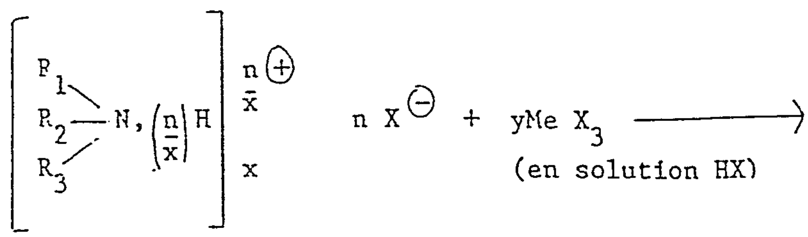


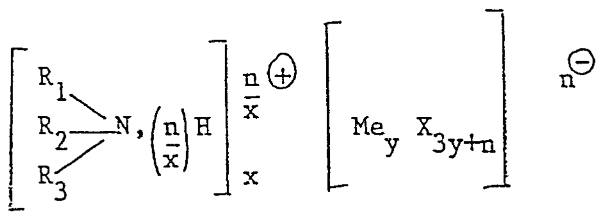

Selon une seconde variante, on fait réagir une amine avec l'halogènure métallique en solution halohydrique contenant un excès d'hydracide selon la réaction :

c) 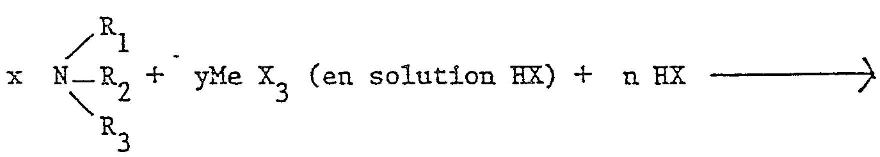


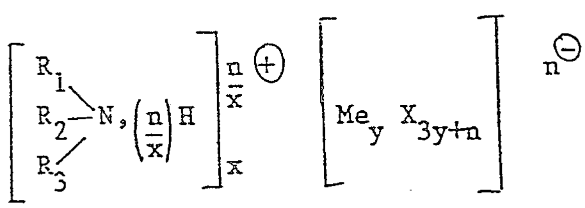

Comme exemples d'amines utilisables, on peut citer à titre non limitatif : la mélamine, l'acétoguanamine, la benzoguanamine, l'amino-3 triazole-1,2,4, le diamino-3,5 triazole-1,2,4 (guanazole), l'amino-2 benzimidazole, le N-aminophtalimide, le N-aminotétrabromophtalimide, le diamino-2,4 nonyl-6 triazine-1,3,5 ; le bis(triazinyl-1,3,5 triamino-2, 4,6) -N, N' éthane-1,2 ; le bis(triazinyl-1,3,5 triamino-2,4,6) -N, N' pipérazine, l'(amino-2 éthyl)-1 pipérazine, la bis(amino-2 éthyl)amine, la pipérazine.

S'agissant de l'une ou l'autre des variantes, on utilise une solution aqueuse d'halogénure métallique préparée en dissolvant un oxyde métallique dans une solution aqueuse concentrée d'hydracide halogéné selon la réaction :

$Me_2 O_3 + 6 HX \rightarrow 2 Me X_3 + 3 H_2 O$ (1)

On opère en utilisant un excès d'hydracide halogéné par rapport à la stoéchiométrie de la réaction (1) compris entre 50 et 100 % molaire et de préférence entre 70 à 80 % (solution S).

Selon la première variante on peut utiliser une solution (ou une suspension) aqueuse concentrée d'halogènure d'ammonium substitué obtenue par addition d'une amine à une solution aqueuse concentrée d'hydracide halogéné de concentration comprise entre 25 et 60 % en poids d'hydracide halogéné et de préférence entre 35 et 50 %.

On opère de préférence en utilisant des quantités stoéchiométriques des réactifs mis en jeu mais il est possible d'utiliser un léger excès d'hydracide halogéné par rapport à l'amine (réaction a).

Selon cette même variante, on peut également utiliser l'halogénure d'ammonium substitué tel quel.

Ensuite on introduit la solution d'halogénure d'ammonium substitué (ou bien l'halogénure d'ammonium substitué tel quel) à la solution d'halogénure métallique S à une température comprise entre 20 et 50° C de préférence entre 30 et 40° C pendant une durée qui peut aller de 1 à plusieurs heures sous bonne agitation.

S'agissant de la seconde variante, on introduit l'amine dans une solution halohydrique d'halogénure métallique $S_1$. Cette solution $S_1$ est préparée en ajoutant à la solution S, la quantité stoéchiométrique d'hydracide halogéné définie selon la réaction c) sous forme d'une solution aqueuse concentrée de cet hydracide halogéné.

L'addition de l'amine s'effectue sous agitation à une température comprise entre 20 et 50° C, de préférence entre 30 et 40° C pendant une durée qui peut aller de 1 à plusieurs heures.

L'addition de l'amine -ou de l'halogénure d'ammonium substitué-terminée, on maintient l'agitation du milieu réactionnel pendant environ une heure à température ambiante (environ 20° C).

Les composés obtenus précipitent généralement au cours de l'addition de l'amine ou de l'halogénure d'ammonium substitué.

Pour les isoler on procède par les moyens connus : concentration de la solution dans le cas où le composé est soluble dans la solution aqueuse halohydrique, filtration, lavage du précipité obtenu par des solvants anhydres tels que les acides carboxyliques (par exemple l'acide acétique ou propionique), les cétones (par exemple l'acétone ou la butanone), les nitriles (par exemple l'acétonitrile), les éthers (par exemple le diéthyléther, le tétrahydrofuranne) ou des mélanges de ceux-ci.

Le produit lavé est essoré puis séché vers 130° C sous pression réduite jusqu'à poids constant. Avantageusement, on broie le produit de façon à obtenir une poudre ayant une granulométrie comprise entre 1 et 50 microns et de préférence entre 5 et 25 microns.

Les produits obtenus sont des poudres plus ou moins colorées en jaune.

L'analyse élémentaire (C, H, N, métal, halogène...) permet de définir les composés obtenus.

Parmi les composés de formule (I) et (I - a) selon l'invention, on préfère utiliser ceux dans lesquels Me représente le bismuth et plus encore l'antimoine, et X représente le brome et le chlore et plus préférentiellement le brome.

On peut citer notamment parmi les composés de formule (I) ceux dans lesquels R, $R_1$, $R_2$ et $R_3$ représentent des atomes hydrogène ; parmi ces composés on peut citer :

- l'hexabromoantimonate (III) de triammonium,
- le nonabromodiantimonate (III) de triammonium,
- l'hexadécabromotriantimonate (III) d'heptaammonium,
- le pentabromobismuthate (III) de diammonium,
- le nonachlorodiantimonate (III) de triammonium.

Parmi les composés de formule (I) dans lesquels R, $R_1$, et $R_2$ sont des hydrogènes, $R_3$ représente un radical hydrocarboné aliphatique linéaire ou ramifié ayant jusqu'à 12 atomes de carbone, un radical cyclohexanique ou un radical phényle, on peut citer : les pentabromoantimonates (III) de bis(n-butylammonium), de bis(isobutylammonium), de bis(tri n-butylammonium), de bis(anilinium), de bis(cyclohexylammonium).

On peut également utiliser les composés de formule I dans lesquels $R_1$, $R_2$ et $R_3$ forment ensemble avec l'atome d'azote un hétérocycle aromatique substitué ou non par des halogènes ou des radicaux aliphatiques linéaires ayant jusqu'à 3 atomes de carbone. Comme hétérocycle aromatique on peut citer la pyridine et dérivés (par exemple les bromo et chloropyridines et les alkylpyridines) et la quinoléine.

Parmi les composés de formule (I) et (I - a) dans lesquels R et $R_1$ représentent des atomes d'hydrogène, $R_2$ et $R_3$ forment ensemble avec l'atome d'azote et le groupement Y un hétérocycle ayant de 2 à 6 atomes de carbone de structure

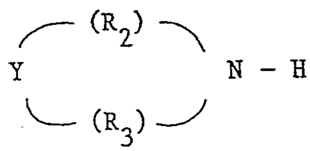

dans lequel Y représente le lien valentiel, H - N$<$ ou $>$N - $(CH_2)_2$-$NH_2$, on peut citer les pentabromoantimonates (III) de bis(pipéridinium), de pipérazinium, d'(ammonio-2 éthyl)-1 pipérazinium, le pentabromobismuthate (III) de pipérazinium et le dodécabromodiantimonate (III) de tris(ammonio-2 éthyl)-1 pipérazinium.

On peut également utiliser avantageusement les composés de formule (I) dans lesquels R, $R_1$ et $R_2$ représentent des atomes d'hydrogène, $R_3$ représente un radical hétérocyclique azoté substitué ou non par des groupements amino, des radicaux aliphatiques linéaires ayant jusqu'à 10 atomes de carbone ou des radicaux phényles. Comme hétérocycles azotés on peut citer les hétérocycles ayant une structure s-triazinique, diazinique-1,3, triazolyle-1,2,4, benzimidazolyle et phtalimidique.

Parmi les composés contenant de tels hétérocycles on peut citer : les pentabromoantimonates (III) de bis(mélaminium), de bis(benzoguanidinium), de bis(acétoguanidinium), de bis(ammonio-2 benzimidazole) et l'hexabromonoantimonate (III) de tris(ammonio-3 triazole-1,2,4).

$R_3$ peut également représenter un reste amino de structure $H_2N$ $(CH_2)_p$- Z-$(CH_2)_m$- , dans lequel Z est une simple liaison ou le groupement $>$N - H et p et m sont identiques et compris entre 1 et 3. On peut citer le pentabromoantimonate (III) de diammonio-1, 2 éthane et l'hexabromoantimonate (III) de bis(ammonio-2 éthyl) ammonium.

L'incorporation des composés de formule I aux matières organiques et notamment aux résines synthétiques permet d'améliorer leur comportement au feu.

Les composés selon l'invention sont utilisés avantageusement entre 1 et 18 % en poids par rapport à la résine à ignifuger, de préférence entre 3 et 15 % et plus préférentiellement entre 5 et 12 %.

Parmi les résines synthétiques que l'on peut ignifuger avec les composés de la présente invention, on peut citer à titre non limitatif : les polyoléfines, les polyamides, les polyesters, les résines vinyliques, les résines ABS, les résines époxy... .

Les compositions ignifugées sont obtenues par incorporation des composés selon l'invention par malaxage des composés finement divisés dans la résine fondue. Tout appareil de malaxage assurant une bonne dispersion peut donc convenir, et en particulier les malaxeurs type BUSS.

Les conditions d'extrusion doivent être appropriées pour obtenir une bonne dispersion des composés, cette démarche relevant de la compétence de l'homme de métier.

Le mélange obtenu est granulé. Les granulés peuvent être moulés par injection ou par compression à des températures convenables en éprouvettes normalisées pour pratiquer l'essai de réaction au feu UL94 selon la norme NF T 51072, pour mesurer l'indice d'oxygène selon la norme NF T 51071 et la résistance au choc IZOD selon la norme ISO 180.

Une manière simple d'opérer consiste à mélanger en présence ou non d'une huile de paraffine, les granulés de polymère et les composés de formule (I) ou (I - a) sous forme de poudre fine dans un mélangeur type TURBULA, et à alimenter avec ce mélange un malaxeur approprié.

On peut également alimenter avec les composés de formule (I) ou (I - a) une extrudeuse au moyen d'un doseur type SODER.

En plus de l'additif ignifugeant, les compositions de résines synthétiques peuvent renfermer également d'autres additifs tels que pigments, colorants, stabilisants vis-à-vis des rayons ultraviolets, agents de démoulage, stabilisants à la dégradation thermique et charges. Ces compositions peuvent renfermer également des fibres de verre dans le but d'améliorer la rigidité de la résine synthétique.

Les exemples suivants explicitent l'invention sans la limiter.

EXEMPLE 1

Préparation du pentabromoantimonate (III) de pipérazinium.

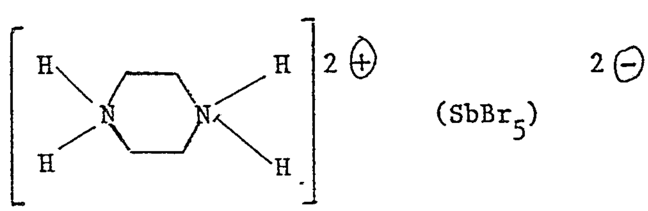

Dans un réacteur de 3 litres équipé d'une agitation à ancre on introduit à 23°C, 1,7 litres d'une solution

d'acide bromhydrique à 48 % (15,3 moles de HBr), puis on introduit par petites portions et sous forte agitation 437,25 g de $Sb_2O_3$ (1,5 moles) en environ 30 minutes.

On obtient une solution limpide à laquelle on ajoute en 3 heures sous forte agitation 743,4 g de dibromure de pipérazinium. La réaction est exothermique et la température s'élève rapidement vers 30-35° C, température que l'on maintient pendant toute la durée de l'addition. Il se forme rapidement un précipité jaune.

L'addition terminée on maintient la suspension sous forte agitation pendant une heure tout en refroidissant le milieu réactionnel de façon à obtenir une température voisine de 20° C.

On essore puis on lave le précipité par de l'acide acétique glacial, on sèche sous pression réduite à 130° C jusqu'à poids constant.

On obtient une poudre jaune.

Analyse élémentaire

| | C | H | N | Sb | Br |
|---|---|---|---|---|---|
| % Calculé | 7,88 | 1,65 | 4,59 | 19,97 | 65,55 |
| % Trouvé | 7,86 | 1,8 | 4,51 | 20,3 | 61,45 |

EXEMPLE 2

Préparation du dodécabromodibismuthate (III) de tris(pipérazinium)

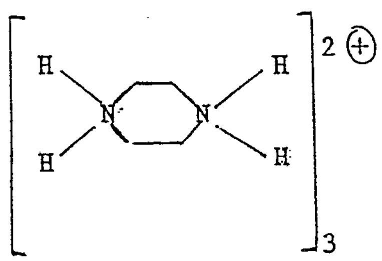

6 ⊖

$(Bi_2\ Br_{12})$

Ce composé est préparé selon la méthode de l'EXEMPLE 1 par introduction de 743,5 g de dibromure de pipérazinium (3 moles) à 2,25 litres d'une solution d'acide bromhydrique à 48 % contenant 898 g de $BiBr_3$ (2 moles).

On opère ensuite comme précédemment.

Le composé précipite pendant l'addition du dibromure de pipérazinium.

Le précipité obtenu est essoré puis lavé par de l'acide acétique glacial et ensuite avec de l'isopropanol.

On sèche sous pression réduite à 130° C jusqu'à poids constant.

On obtient une poudre jaune.

Analyse élémentaire

| | C | H | N | Bi |
|---|---|---|---|---|
| % Calculé | 8,77 | 2,19 | 5,12 | 25,46 |
| % Trouvé | 8,6 | 2,15 | 4,77 | 24,3 |

EXEMPLE 3

Préparation du pentabromoantimonate (III) de bis(mélaminium).

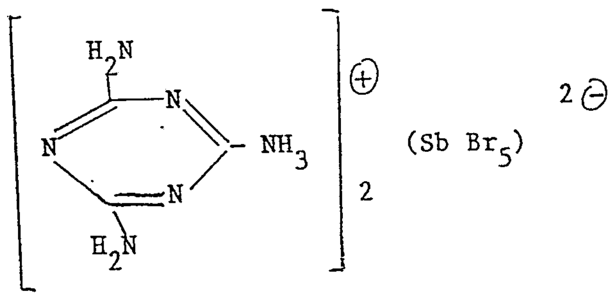

A une solution bromhydrique de tribromure d'antimoine préparée comme dans l'EXEMPLE 1 et contenant 361,45 g de tribromure d'antimoine (1 mole) on ajoute 223 ml d'une solution d'acide bromhydrique à 48 % soit 2 moles de HBr.

A cette solution on ajoute sous forte agitation 252 g de mélamine (2 moles) en 2 heures.

Il se forme rapidement un précipité jaune. On maintient la température vers 30-35° C pendant toute la durée de l'addition puis celle-ci terminée on refroidit vers 20° C, toujours sous forte agitation.

On essore, puis on lave le gâteau obtenu avec de l'acide acétique glacial.

On sèche sous pression réduite à 130° C jusqu'à poids constant.

On obtient une poudre jaune pâle.

Analyse élémentaire

$C_6 H_{14} N_{12} SbBr_5$ (Masse Moléculaire : 775,25)

| | C | H | N | Br | Sb |
|---|---|---|---|---|---|
| % Calculé | 9,29 | 1,81 | 21,67 | 51,53 | 15,70 |
| % Trouvé | 9,09 | 1,57 | 21 | 50,35 | 16,27 |

EXEMPLE 4

Préparation du pentabromoantimonate (III) de bis(benzoguanaminium)

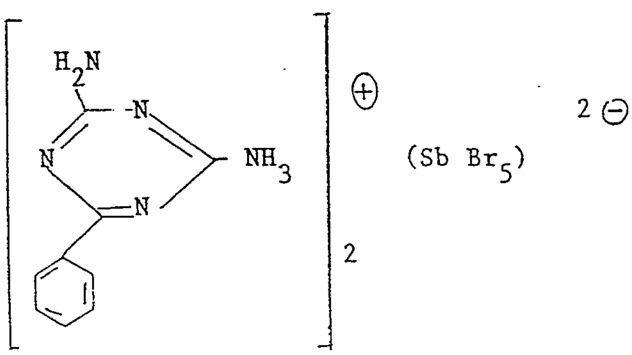

On opère comme dans l'EXEMPLE 3 mais en remplaçant la mélamine par 374 g de benzoguanamine (2 moles).

Le produit obtenu est une poudre jaune.

Analyse élémentaire

| | C | H | N | Br | Sb |
|---|---|---|---|---|---|
| % Calculé | 24,08 | 2,25 | 15,6 | 44,50 | 13,56 |
| % Trouvé | 24 | 2,15 | 15,6 | 46,3 | 11,3 |

EXEMPLE 5

Préparation du pentabromoantimonate (III) de bis(ammonio-2 benzimidazole)

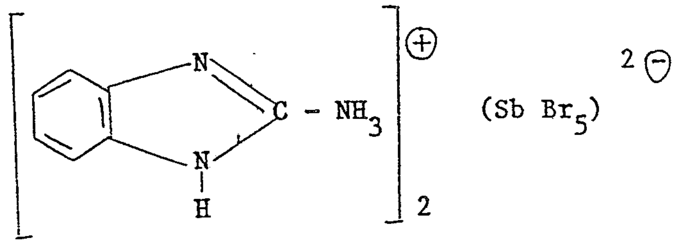

On opère comme dans l'EXEMPLE 3 mais en remplaçant la mélamine par 266 g de N-amino benzimidazole (2 moles).

Le produit obtenu est une poudre gris-violet.

Analyse élémentaire

| | C | H | N | Br | Sb |
|---|---|---|---|---|---|
| % Calculé | 21,3 | 2 | 10,64 | 50,6 | 15,42 |
| % Trouvé | 21,9 | 1,52 | 10,4 | 50,15 | 15,2 |

EXEMPLE 6

Préparation de l'hexabromoantimonate (III) de tris(ammonio-3 triazole-1,2,4)

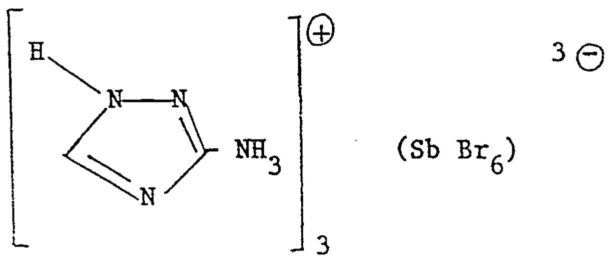

On opère comme dans l'EXEMPLE 3 mais en remplaçant la mélamine par 252 g d'amino-3 triazole-1,2,4 (3 moles) et en utilisant 334,5 ml d'une solution d'acide bromhydrique à 48 % (3 moles d'HBr) au lieu de 223 ml.

L'addition terminée, on concentre partiellement la solution obtenue puis on essore. On procède ensuite comme précédemment.

Le produit obtenu se présente sous forme d'une poudre jaune pâle qui commence à se ramollir vers 50° C.

Analyse élémentaire

| | C | H | N | Br | Sb |
|---|---|---|---|---|---|
| % Calculé | 8,41 | 1,77 | 19,63 | 56 | 14,22 |
| % Trouvé | 8,3 | 1,75 | 20 | 55,9 | 14,7 |

EXEMPLE 7

Préparation de pentabromoantimonate (III) d'(ammonio-2 éthyl)-1 pipérazinium

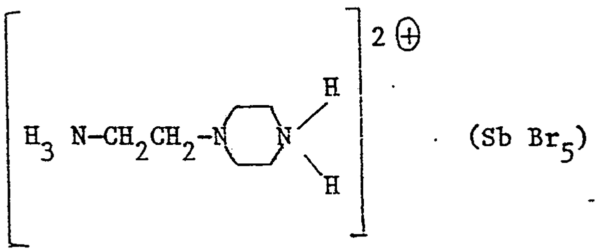

EMI ID=13/2 HE=40 WI=90 TI=CHE

On opère comme dans l'EXEMPLE 3 mais en remplaçant la mélamine par 129 g d'amino-2 éthyl pipérazine (1 mole).

Le produit obtenu est une poudre jaune.

Analyse élémentaire

| | C | H | N | Br | Sb |
|---|---|---|---|---|---|
| % Calculé | 11,04 | 2,31 | 6,44 | 61,22 | 18,66 |
| % Trouvé | 9,74 | 2,48 | 5,69 | 61,3 | 16,5 |

EXEMPLE 8

Préparation du dodécabromodiantimonate (III) de tris(ammonio-2 éthyl)-1 pipérazinium

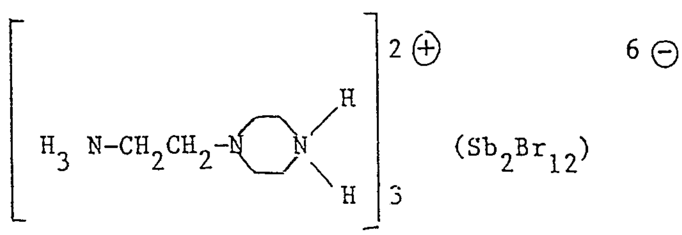

On opère comme dans l'EXEMPLE 3 mais en remplaçant la mélamine par 193,5 g d'amino-2 éthyl pipérazine (1,5 moles) et en utilisant 334,5 ml d'une solution d'acide bromhydrique à 48 % (3 moles d'HBr) au lieu de 223 ml.

Le produit obtenu est une poudre jaune.

Analyse élémentaire

| | C | H | N | Br | Sb |
|---|---|---|---|---|---|
| % Calculé | 13,54 | 2,84 | 7,89 | 60,07 | 15,26 |
| % Trouvé | 10,88 | 2,57 | 6,16 | 63 | 14,77 |

EXEMPLE 9

Hexabromoantimonate (III) de bis(ammonio-2 éthyl)ammonium

$$\left[(H_3N{-}CH_2CH_2)_2\ NH_2\right]^{3\oplus} \qquad (Sb\ Br_6)^{3\ominus}$$

On opère comme dans l'EXEMPLE 3 mais en remplaçant la mélamine par 103 g de bis(amino-2 éthyl)amine (1 mole) et un utilisant 334,5 ml d'une solution d'acide bromhydrique à 48 % (3 moles d'HBr) au lieu de 223 ml.

Le produit obtenu est une poudre jaune.

Analyse élémentaire

| | C | H | N | Sb | Br |
|---|---|---|---|---|---|
| % Calculé | 6,78 | 2,26 | 5,94 | 17,21 | 67,76 |
| % Trouvé | 7,15 | 2,35 | 6,3 | 18,9 | 67,9 |

EXEMPLE 10

Des granulés de polypropylène de densité = 0,905 g/cm³ et de melt index 5 (charge 2,16 kg à 230°C) sont extrudés sur malaxeur BUSS, type PR 46, dont la température moyenne est de 200-210°C.

Les granulés obtenus sont moulés par injection en éprouvettes normalisées sur lesquelles on pratique l'essai UL94 selon la norme NF T 51072 (éprouvette verticale), la mesure de l'indice d'oxygène (norme NF T 51071) et la mesure du choc IZOD selon la norme ISO 180 (éprouvette entaillée).

EXEMPLE 11

On mélange (mélangeur TURBULA) :

- 8 980 g de granulés de polypropylène
- 20 g d'une huile de paraffine codex
- 1 000 g d'hexabromoantimonate (III) de triammonium.

On alimente avec ce mélange un malaxeur BUSS type PR 46 dont la température moyenne est de 200°C.

Les granulés obtenus sont moulés par injection en éprouvettes normalisées sur lesquelles on pratique l'essai UL 94, la mesure de l'indice d'oxygène et du choc IZOD.

EXEMPLE 12

Analogue à l'EXEMPLE 11 sauf qu'on remplace l'hexabromoantimonate (III) de triammonium par la même quantité pondérale de nonabromodiantimonate (III) de triammonium.

EXEMPLE 13

Analogue à l'EXEMPLE 11 sauf qu'on remplace l'hexabromoantimonate (III) de triammonium par une quantité deux fois moins importante d'hexadécabromotriantimonate (III) d'heptaammonium.

EXEMPLES 14 et 15

Analogues à l'EXEMPLE 13 sauf qu'on utilise des doses différentes d'hexadécabromotriantimonate (III) d'heptaammonium.

EXEMPLE 16

Analogue à l'EXEMPLE 11 sauf qu'on remplace l'hexabromoantimonate (III) de triammonium par la même quantité pondérale de pentabromobismuthate (III) de diammonium.

EXEMPLES 17 à 25

Les exemples 17 à 25 sont analogues à l'EXEMPLE 11 sauf qu'on remplace l'hexabromoantimonate (III) de triammonium par la même quantité pondérale des produits suivants :

EXEMPLE 17 : pentabromoantimonate (III) de bis(mélaminium)
EXEMPLE 18 : pentabromoantimonate (III) de bis(pipéridinium)
EXEMPLE 19 : pentachloroantimonate (III) de bis(pipéridinium)
EXEMPLE 20 : pentabromoantimonate (III) de bis(anilinium)
EXEMPLE 21 : pentabromoantimonate (III) de pipérazinium
EXEMPLE 22 : pentabromoantimonate (III) d'(ammonio-2 éthyl)-1 pipérazinium
EXEMPLE 23 : dodécabromodiantimonate (III) de tris(ammonio-2 éthyl)-1 pipérazinium
EXEMPLE 24 : pentabromoantimonate (III) de diammonio-1,2 éthane
EXEMPLE 25 : dodécabromodibismuthate (III) de tris(pipérazinium).

EXEMPLE 26

Cet exemple, dans lequel on utilise des produits commerciaux couramment employés pour améliorer le comportement au feu du polypropylène est donné à titre comparatif :

On mélange à sec au Turbula :

- 6 270 g de polypropylène
- 2 800 g de décabromobiphényle
- 930 g de $Sb_2O_3$.

On alimente avec ce mélange un malaxeur BUSS Type PR 46.

On procède ensuite comme dans l'EXEMPLE 17.

Les résultats obtenus sont reportés dans le Tableau A.

TABLEAU A

| FORMULATIONS \ EXEMPLES | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|
| Polypropylène | 100 | 89,8 | 89,8 | 94,8 | 89,8 | 87,8 | 89,8 | 89,8 | 89,8 | 89,8 | 89,8 | 89,8 | 89,8 | 89,8 | 89,8 | 89,8 | 62,7 |
| Huile de paraffine Codex | | 0,2 | 0,2 | 0,2 | 0,2 | 0,2 | 0,2 | 0,2 | 0,2 | 0,2 | 0,2 | 0,2 | 0,2 | 0,2 | 0,2 | 0,2 | |
| $(NH_4)_3^{\oplus} (SbBr_6)^{3\ominus}$ | | 10 | | | | | | | | | | | | | | | |
| $(NH_4)_3^{\oplus} (Sb_2Br_9)^{3\ominus}$ | | | 10 | | | | | | | | | | | | | | |
| $(NH_4)_7^{\oplus} (Sb_3Br_{16})^{7\ominus}$ | | | | 5 | 10 | 12 | | | | | | | | | | | |
| $(NH_4)_2^{\oplus} (BiBr_5)^{2\ominus}$ | | | | | | | 10 | | | | | | | | | | |
| pentabromoantimonate (III) de bis(mélaminium) | | | | | | | | 10 | | | | | | | | | |
| pentabromoantimonate (III) de bis(pipéridinium) | | | | | | | | | 10 | | | | | | | | |
| pentachloroantimonate(III) de bis(pipéridinium) | | | | | | | | | | 10 | | | | | | | |
| pentabromoantimonate (III) de bis(anilinium) | | | | | | | | | | | 10 | | | | | | |
| pentabromoantimonate (III) de pipérazinium | | | | | | | | | | | | 10 | | | | | |

TABLEAU A (SUITE)

| FORMULATIONS \ EXEMPLES | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|
| pentabromoantimonate (III) d'(ammonio-2 éthyl)-1 pipérazinium | | | | | | | | | | | | | 10 | | | | |
| dodécabromodiantimonate (III) de tris(ammonio-2 éthyl)-1 pipérazinium | | | | | | | | | | | | | | 10 | | | |
| pentabromoantimonate (III) de diammonio-1,2 éthane | | | | | | | | | | | | | | | 10 | | |
| dodécabromodibismuthate (III) de tris(pipérazinium) | | | | | | | | | | | | | | | | 10 | |
| décabromobiphényle | | | | | | | | | | | | | | | | | 28 |
| $Sb_2O_3$ | | | | | | | | | | | | | | | | | 9,3 |
| Indice d'oxygène (%) | 17 | 25,4 | 25,2 | 22,8 | 26,5 | 25,8 | 25,8 | 25,5 | 27,4 | 22 | 25,7 | 26,7 | 28,4 | 28,3 | 26,8 | 26,1 | 27,7 |
| ESSAI UL94 : Classement | | | | | | | | | | | | | | | | | |
| Epaisseur 3,2 mm | NC | V2 | V2 | V2 | VO | VO | V2 | V2 | V2 | V2 | V2 | V2 | V2 | VO | V2 | V2 | NC |
| Epaisseur 1,6 mm | NC | V2 | | V2 | V2 | VO | V2 | | | | | | | VO | | | |
| Choc IZOD (Kj/m²) | 3,64 | 3,3 | 3 | | 3,2 | | | 3,02 | | | | | | 3,11 | 3,5 | | 2,55 |

La supériorité des composés selon l'invention sur ceux de la technique antérieure utilisés à des taux élevés est bien nette. En effet, des quantités trois fois moins importantes permettent d'obtenir un niveau d'ignifugation élevé tout en conservant un niveau de résistance au choc supérieur.

EXEMPLE 27

Des granulés de polyamide 11 présentant les caractéristiques suivantes : viscosité de solution = 1,01, densité = 1,03, température de fusion = 185°C sont extrudés sur malaxeur BUSS, type PR46. Les granulés obtenus sont moulés par injection en éprouvettes normalisées sur lesquelles on pratique l'essai UL94 et la mesure de l'indice d'oxygène.

EXEMPLE 28

On mélange :

- 9480 g de granulés de polyamide 11 ayant les mêmes caractéristiques que dans l'exemple 27
- 20 g d'une huile de paraffine Codex puis après un temps de mélange nécessaire pour disperser l'huile de paraffine on ajoute :
- 500 g de $(NH_4)_3{}^{\oplus}\ (SbBr_6)^{3\ominus}$

On alimente avec ce mélange un malaxeur BUSS type PR46.

Les granulés obtenus sont moulés par injection en éprouvettes normalisées sur lesquelles on pratique l'essai UL94 et la mesure de l'indice d'oxygène.

EXEMPLE 29

Analogue à l'exemple 28 sauf qu'on remplace le $(NH_4)_3{}^{\oplus}\ (SbBr_6)^{3\ominus}$ par la même quantité pondérale de $(NH_4)_3{}^{\oplus}\ (Sb_2Br_9)^{3\ominus}$.

EXEMPLE 30

Analogue à l'exemple 28 sauf qu'on remplace la $(NH_4)_3{}^{\oplus}\ (SbBr_6)^{3\ominus}$ par la même quantité pondérale de $(NH_4)_7{}^{\oplus}\ (Sb_3Br_{16})^{7\ominus}$.

EXEMPLE 31

Analogue à l'exemple 30 mais avec une quantité de $(NH_4)_7{}^{\oplus}\ (Sb_3Br_{16})^{7\ominus}$ différente.

EXEMPLES 32, 33 et 34

Analogues à l'exemple 28 sauf qu'on remplace le composé $(NH_4)_3{}^{\oplus}\ (SbBr_6)^{3\ominus}$ par les composés suivants :

EXEMPLE 32 pentabromoantimonate (III) de bis(mélaminium)
EXEMPLE 33 pentabromoantimonate (III) de diammonio-1,2 éthane
EXEMPLE 34 pentabromoantimonate (III) de bis(pipéridinium)

EXEMPLE 35

Cet exemple dans lequel on utilise des produits commerciaux couramment employés pour améliorer le comportement au feu du Polyamide 11 est donné à titre comparatif.

On mélange à sec au Turbula :

- 9550 g de PA 11
- 300 g de décabromobiphényle
- 150 g de $Sb_2O_3$

On alimente avec ce mélange un malaxeur BUSS PR46.

On procède ensuite comme dans l'exemple 27.

Les résultats sont rassemblés dans le tableau B.

TABLEAU B

| FORMULATION \ EXEMPLES | 27 | 28 | 29 | 30 | 31 | 32 | 33 | 34 | 35 |
|---|---|---|---|---|---|---|---|---|---|
| Polyamide 11 | 100 | 94,8 | 94,8 | 94,8 | 96,8 | 94,8 | 94,8 | 94,8 | 95,5 |
| Huile de paraffine Codex | - | 0,2 | 0,2 | 0,2 | 0,2 | 0,2 | 0,2 | 0,2 | - |
| $(NH_4)_3^{\oplus}(SbBr_6)^{3\ominus}$ | | 5 | | | | | | | |
| $(NH_4)_3^{\oplus}(Sb_2Br_9)^{3\ominus}$ | | | 5 | | | | | | |
| $(NH_4)_7^{\oplus}(Sb_3Br_{16})^{7\ominus}$ | | | | 5 | 3 | | | | |
| Pentabromoantimonate (III) de bis(mélaminium) | | | | | | 5 | | | |
| Pentabromoantimonate(III) de diammonio-1,2 éthane | | | | | | | 5 | | |
| Pentabromoantimonate (III) de bis(pipéridinium) | | | | | | | | 5 | |
| décabromobiphényle | | | | | | | | | 3 |
| $Sb_2O_3$ | | | | | | | | | 1,5 |
| Indice d'oxygène (%) | 22,8 | 35,1 | 34,8 | 36,5 | 36,9 | 31,9 | 33,1 | 33,3 | 31,4 |
| ESSAI UL94 | | | | | | | | | |
| Epaisseur 3,2mm Classement | V2 | VO | VO | VO | VO | VO | VO | VO | V2 |
| Epaisseur 1,6mm Classement | V2 | VO | VO | VO | VO | VO | VO | VO | |

EXEMPLE 36

Des granulés de polyéthylène basse densité de densité 0,91 g/cm³ sont extrudés sur malaxeur BUSS, type PR46.

Les granulés obtenus sont moulés par injection en éprouvettes normalisées sur lesquelles on pratique l'essai UL94 et la mesure de l'indice d'oxygène.

EXEMPLE 37

On mélange au Turbula :

- 8480 g de polyéthylène basse densité
- 20 g d'une huile de paraffine
- 1500 g de $(NH_4)_7^{\oplus} (Sb_3Br_{16})^{7\ominus}$

On alimente avec ce mélange un malaxeur BUSS type PR46.

Les granulés obtenus sont moulés par injection en éprouvettes normalisées sur lesquelles on pratique l'essai UL94 et la mesure de l'indice d'oxygène.

Les exemples 38 et 39 concernent le copolymère d'éthylène-acétate de vinyle.

Les exemples 40 et 41 concernent le polybutylène téréphtalate.

Les exemples 42 et 43 concernent les résines ABS.

Les résultats des exemples 36 à 43 sont rassemblés dans le tableau C.

TABLEAU C

| FORMULATION \ EXEMPLES | 36 | 37 | 38 | 39 | 40 | 41 | 42 | 43 |
|---|---|---|---|---|---|---|---|---|
| polyéthylène basse densité | 100 | 84,8 | | | | | | |
| copolymère éthylène Acétate de vinyle | | | 100 | 87,3 | | | | |
| polybutylène téréphtalate | | | | | 100 | 89,8 | | |
| résine ABS | | | | | | | 100 | 89,8 |
| $(NH_4)_7^{\oplus} (Sb_3Br_{16})^{7\ominus}$ | | 15 | | | | 10 | | 10 |
| $(NH_4)_2^{\oplus} (BiBr_5)^{2\ominus}$ | | | | 12,5 | | | | |
| Huile de paraffine Codex | | 0,2 | | 0,2 | | 0,2 | | 0,2 |
| Indice d'oxygène (%) | 18 | 24,9 | 21,1 | 24,2 | 21,6 | 31,4 | 19,7 | 21,5 |
| ESSAI UL94 | | | | | | | | |
| Epaisseur 3,2 mm Classement | NC | V2 | NC | NC | NC | V0 | NC | NC |
| Epaisseur 1,6 mm Classement | | V2 | | | | V0 | | |

Exemple 44

On mélange (mélangeur Turbula)

- 8680 g de granulés de polypropylène
- 20 g d'une huile de paraffine Codex
- 1300 g de tétrabromoantimonate (III) de tétrabutylammonium

$[(_n Bu)_4 N]^{\oplus} (SbBr_4)^{\ominus}$

On alimente avec ce mélange un malaxeur BUSS PR 46 dont la température moyenne est de 200°C. Les granulés obtenus sont moulés par injection en éprouvettes normalisées sur lesquelles on pratique l'essai UL 94 et la mesure de l'indice d'oxygène.

Exemple 45

Analogue à l'exemple 44 sauf qu'on utilise une dose différente de tétrabromoantimonate (III) de tétrabutylammonium.

Les résultats obtenus sont reportés dans le Tableau D.

TABLEAU D

| FORMULATION / EXEMPLES | 44 | 45 |
|---|---|---|
| Polypropylène | 86,80 | 83,20 |
| Huile de paraffine Codex | 0,20 | 0,20 |
| $[(_n Bu)_4 N]^{\oplus} (SbBr_4)^{\ominus}$ | 13 | 16,60 |
| Indice d'oxygène (%) | 27,3 | 27,6 |
| ESSAI UL 94 : Classement | | |
| Epaisseur 3,2 mm | V2 | V2 |
| Epaisseur 1,6 mm | V2 | V2 |

**Revendications**

1.- Compositions ignifugeantes de résines synthétiques, caractérisées en ce qu'elles comprennent au moins un composé organométallique renfermant des groupements cationiques azotés et des groupements anioniques halogéno-métalliques répondant à la formule générale

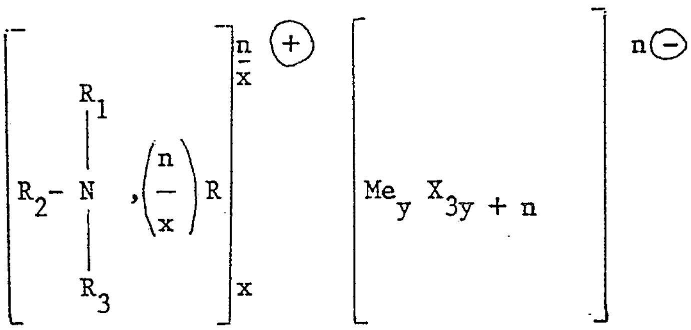

I

dans laquelle :
R, $R_1$, $R_2$ et $R_3$, identiques ou différents, représentent des atomes d'hydrogène, des radicaux hydrocarbonés aliphatiques linéaires ou ramifiés, éventuellement substitués par des halogènes et ayant jusqu'à 12 atomes de carbone, des radicaux cycloaliphatiques, des radicaux phényles substitués ou non par des halogènes, des groupements amino, des groupements aliphatiques linéaires ou ramifiés ayant jusqu'à 6 atomes de carbone ;
$R_1$, $R_2$ et $R_3$ peuvent former ensemble avec l'atome d'azote un hétérocycle aromatique substitué ou non par des groupements aliphatiques linéaires ou ramifiés ayant jusqu'à 6 atomes de carbone ou des halogènes ;
$R_2$ et $R_3$ peuvent également former ensemble avec l'atome d'azote et le groupement divalent (Y) un hétérocycle ayant de 2 à 10 atomes de carbone de formule :

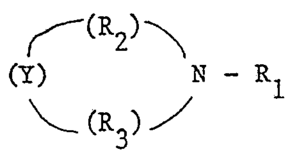

dans laquelle (Y) peut représenter le lien valentiel,

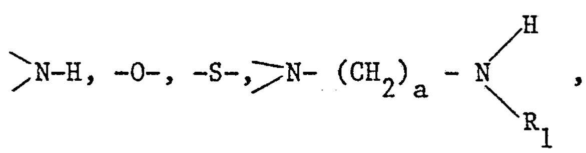

avec a compris entre 2 et 6, $R_1$ ayant la signification donnée précédemment ;
$R_3$ peut représenter un radical hétérocyclique azoté substitué ou non par des halogènes, des groupements amino, des radicaux aliphatiques linéaires ou ramifiés ayant jusqu'à 10 atomes de carbone, des radicaux phényles ou un reste amino de formule

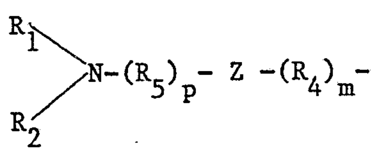

dans laquelle $R_1$ et $R_2$ ont la signification donnée précédemment, $R_4$ et $R_5$, identiques ou différents, représentent des radicaux divalents hétérocycliques azotés ou bien des restes méthyléniques,
Z représente une simple liaison ou un groupement divalent pris parmi les suivants :

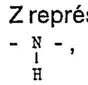

- NH - $(CH_2)_b$ NH - avec b compris entre 0 et 6, - $(CH_2)_c$ avec c compris entre 1 et 6 et

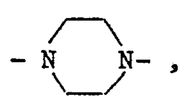

p et m sont compris entre 1 et 6.
Me représente un métal tel que l'arsenic (III), l'antimoine (III) ou le bismuth (III) ;
X représente un halogène tel que le chlore, le brome ou l'iode

x, y et n sont des nombres entiers compris entre 1 et 10 avec n ≧ x

2.- Compositions selon la revendication 1, caractérisées en ce que dans les produits de formule I, R, $R_1$, $R_2$ et $R_3$ représentent des atomes d'hydrogène, Me représente l'antimoine (III) ou le bismuth (III) et plus préférentiellement l'antimoine (III), X représente le brome ou le chlore.

3.- Compositions selon l'une quelconque des revendications 1 ou 2, dans lesquelles les produits sont choisis dans le groupe constitué par : l'hexabromoantimonate (III) de triammonium, le nonabromodiantimonate (III) de triammonium, l'hexadécabromotriantimonate (III) d'heptaammonium, le nonabromodibismuthate (III) de triammonium, le nonachlorodiantimonate (III) de triammonium.

4.- Compositions selon la revendication 1, caractérisées en ce que dans les produits de formule I, R, $R_1$ et $R_2$ représentent des atomes d'hydrogène, $R_3$ représente un radical hydrocarboné aliphatique linéaire ou ramifié ayant jusqu'à 12 atomes de carbone, un radical cyclohexanique, un radical phényle, Me et X sont tels que définis dans la revendication 2.

5.- Compositions selon l'une quelconque des revendications 1 ou 4 dans lesquelles les produits sont choisis dans le groupe constitué par : le pentabromoantimonate (III) de bis(n-butylammonium), le pentabromoantimonate (III) de bis(isobutylammonium), le pentabromoantimonate (III) de bis-(tri n-butylammonium), le pentabromoantimonate (III) de bis(anilinium), le pentabromoantimonate (III) de bis(cyclohexylammonium).

6.- Compositions selon la revendication 1, caractérisées en ce que dans les produits de formule I, R représente un atome d'hydrogène, $R_1$, $R_2$ et $R_3$ forment ensemble avec l'atome d'azote un hétérocycle aromatique substitué ou non par des radicaux aliphatiques linéaires ayant jusqu'à 3 atomes de carbone ou des halogènes, Me et X sont tels que définis dans la revendication 2.

7.- Compositions selon la revendication 1, caractérisées en ce que dans les produits de formule I, R et $R_1$ représentent des atomes d'hydrogène, $R_2$ et $R_3$ forment ensemble avec l'atome d'azote et le groupement divalent (Y) un hétérocycle ayant de 2 à 6 atomes de carbone de structure

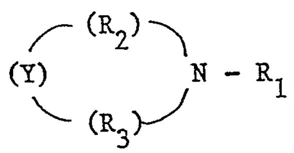

dans lequel (Y) représente le lien valentiel, >N - H et >N - $(CH_2)$-$NH_2$, Me et X sont tels que définis dans la revendication 2.

8.- Compositions selon l'une quelconque des revendications 1 ou 7, dans lesquelles les produits sont choisis dans le groupe constitué par : le pentabromoantimonate (III) bis(pipéridinium), le pentabromoantimonate (III) de pipérazinium, le pentabromobismuthate (III) de pipérazinium, le pentabromoantimonate (III) d'(ammonio-2 éthyl)-1 pipérazinium, le dodécabromodiantimonate (III) de tris(ammonio-2 éthyl)-1 pipérazinium.

9.- Compositions selon la revendication 1, caractérisées en ce que dans les produits de formule I, R, $R_1$ et $R_2$ représentent des atomes d'hydrogène, $R_3$ représente un radical hétérocyclique azoté substitué ou non par des groupements amino, des radicaux aliphatiques linéaires ou ramifiés ayant jusqu'à 10 atomes de carbone ou des radicaux phényles, Me et X sont tels que définis dans la revendication 2.

10.- Compositions selon l'une quelconque des revendications 1 ou 9, caractérisées en ce que dans les produits de formule I, $R_3$ est un radical hétérocyclique choisi parmi les radicaux de structure s-triazinique, diazinique-1,3, triazolyle-1,2,4, benzimidazolyle et phtalimidique.

11.- Composition selon la revendication 10, dans laquelle le produit de formule I est le pentabromoantimonate (III) de bis(mélamimium).

12.- Compositions selon la revendication 1, caractérisées en ce que dans les produits de formule I, R, $R_1$ et $R_2$ représentent des atomes d'hydrogène, $R_3$ un reste amino de structure $H_2N$-$(R_5)$p-Z-$(R_4)_m$- dans lequel $R_4$ et $R_5$ représentent des restes méthyléniques ou des radicaux divalents s-triazinique, Z est une simple liaison ou un groupement divalent pris parmi les suivants :

$-(CH_2)_2-$ et 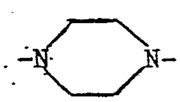 ,

p et m sont compris entre 1 et 4, Me et X sont tels que définis dans la revendication 2.

13.- Compositions selon l'une quelconque des revendications 1 ou 12 caractérisées en ce que dans les produits de formule I $R_3$ est un radical amino de structure $H_2N(CH_2)_p$-$Z(CH_2)_m$- dans lequel Z est une simple liaison ou le groupement divalent >N - H et m = p et est compris entre 1 et 3.

14.- Composition selon la revendication 13, dans laquelle le produit de formule I est le pentabromoantimonate (III) de diammonio-1,2 éthane ou l'hexabromoantimonate (III) de bis(ammonio-2 éthyl)ammonium.

15.- Les composés organométalliques renfermant des groupements cationiques azotés et des groupements anioniques halogèno-métalliques de formule :

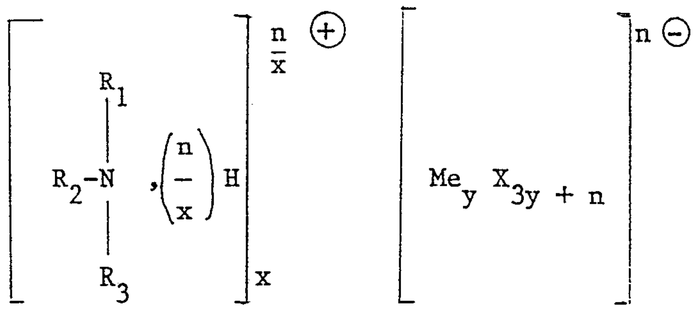

(I - a)

dans laquelle $R_1$ et $R_2$, identiques ou différents, représentent des atomes d'hydrogène, des radicaux aliphatiques linéaires ou ramifiés, ayant jusqu'à 12 atomes de carbone, des radicaux phényles ;
$R_3$ représente un radical hétérocyclique azoté C-substitué ou non par des halogènes, des groupements amino, des radicaux aliphatiques linéaires ou ramifiés ayant jusqu'à 10 atomes de carbone, des radicaux phényles ou un reste amino de structure $H_2N\ -(R_5)_p-Z(R_4)$ dans lequel $R_4$ et $R_5$ identiques ou différents, représentent des radicaux azotés divalents hétérocycliques, Z est un groupement divalent pris parmi les suivants :
$>N-H$, $-NH(CH_2)_b-NH-$ avec b compris entre 0 et 6,

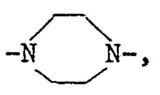

ou un reste $-(CH_2)_c-$avec c compris entre 1 et 6 lorsque $R_4$ et $R_5$ représentent des radicaux divalents hétérocycliques, p et m sont compris entre 1 et 6 ;
$R_2$ et $R_3$ peuvent former également ensemble avec l'atome d'azote et les groupements divalents $>N-H$ et $>N-(CH_2)_a-NH_2$ avec a compris entre 2 et 6, un hétérocycle ayant de 2 à 10 atomes de carbone ; Me, X, n, x et y sont tels que définis dans la revendication 1.

16.- Composés selon la revendication 15, dans lesquels $R_1$ et $R_2$ représentent des atomes d'hydrogène, Me représente le bismuth et plus encore l'antimoine, X représente le brome et le chlore et plus préférentiellement le brome, $R_3$ est un radical hétérocyclique pris parmi les radicaux de structure s-triazinique, diazinique-1,3, triazolyle-1,2,4, benzimidazolyle et phtalimidique.

17.- Le pentabromoantimonate (III) de bis(mélaminium).

18.- Le pentabromoantimonate (III) de bis(benzoguanidinium).

19.- L'hexabromoantimonate (III) de tris(ammonio-3 triazole-1,2,4)

20.- Composés selon la revendication 15 dans lesquels $R_1$ représente un atome d'hydrogène, $R_2$ et $R_3$ forment avec l'atome d'azote et les groupements $>N-H$ ou $>N-(CH_2)_2-NH_2$ un hétérocycle à structure pipérazinique.

21.- Le pentabromoantimonate (III) de pipérazinium.

22.- Le dodécabromodibismuthate (III) de tris(pipérazinium).

23.- Le pentabromoantimonate (III) d'(ammonio-2 éthyl)-1 pipérazinium.

24.- Le dodécabromodiantimonate (III) de tris(ammonio-2 éthyl)-1 pipérazinium.

25.- Une composition ignifugée de résine synthétique caractérisée en ce qu'elle comprend une composition ignifugeante selon l'une quelconque des revendications 1 à 24.

26.- Composition selon la revendication 25 caractérisée en ce qu'elle comprend 1 à 18 % en poids de produit de formule (I) ou (I - a) par rapport au poids de résine synthétique.